# EUROPEAN PATENT APPLICATION

(11) **EP 4 806 376 A1**
(43) Date of publication of application: **16.09.2026**
(21) Application number: 23957883.4
(22) Date of filing: 06.11.2023
(51) Int. Cl.: A61B 18/22

(54) **OPTICAL FIBER COMBINING-COUPLED ULTRAVIOLET LASER ABLATION SYSTEM**

(71) Applicant: Micro-Energy Medical Technology Co., Ltd., Shenzhen, Guangdong 518118 (CN)
(72) Inventor: HUANG, Zhisheng, Shenzhen, Guangdong 518118 (CN); AGANOGLU, Ruzin, Shenzhen, Guangdong 518118 (CN); KUFNER, Stefan, Shenzhen, Guangdong 518118 (CN); WU, Nan, Shenzhen, Guangdong 518118 (CN); WANG, Gang, Shenzhen, Guangdong 518118 (CN)
(74) Representative: Metida
(86) International application number: PCT/CN2023/129838
(87) International publication number: WO 2025/097269

(57) **Abstract**

The present invention discloses an optical fiber combining-coupled ultraviolet laser ablation system, which relates to the field of medical devices. The system comprises a laser, a light homogenizing assembly, and an optical fiber combining assembly; the laser is configured to emit laser light; the light homogenizing assembly is disposed on the emergent light path of the laser light; the optical fiber combining assembly is disposed on the emergent light path of the light homogenizing assembly; the light homogenizing assembly is used for performing beam flat-top shaping and beam superposition on the laser light to obtain a target light spot; the optical fiber combining assembly is used for performing multi-beam transmission on the target light spot. The present invention can realize efficient and stable laser ablation.

## Description

### TECHNICAL FIELD

The present invention relates to the field of medical devices, and more particularly to an optical fiber combining-coupled ultraviolet laser ablation system.

### BACKGROUND

For conditions such as chronic total occlusion lesions, thrombi, and concurrent atheromatous plaques and calcified tissue lesions within blood vessels, endovascular interventional therapy is generally employed. A medical catheter is delivered into the blood vessel, and laser ablation principle is used to eliminate plaques and hyperplastic tissues, thereby recanalizing occluded or stenotic blood vessels and achieving safe treatment without damaging the blood vessels.

When laser interacts with biological tissues, a series of biological effects are generated, including photochemical effect, thermal effect, and mechanical effect. High-peak-power pulsed ultraviolet (UV) lasers with a pulse width in the nanosecond range have high photon energy, which can directly break chemical bonds between molecules of biological tissues and fragment macromolecular compounds into small debris (with a size of less than 20 µm). These small debris are ultimately absorbed by the reticuloendothelial system, thereby avoiding microvascular occlusion. When 355 nm UV laser is used for ablating thrombi and other lesion tissues, the photochemical effect plays a dominant role, while the thermal effect is minimal.

Currently, the diameter of UV laser ablation catheters is relatively small, and the number and area of ablation optical fibers that can be accommodated within the catheter are limited. This not only restricts the laser energy acceptable at the fiber input end but also easily damages the fiber end face, resulting in unsatisfactory tissue ablation effects. Therefore, it is crucial to improve optical fiber coupling efficiency, output stable laser energy, and prevent damage to the fiber input end face.

### SUMMARY

The objective of the present invention is to provide an optical fiber combining-coupled ultraviolet laser ablation system, so that laser ablation can be efficiently and stably achieved.

To achieve the above objective, the present invention provides the following technical solution:
An optical fiber combining-coupled ultraviolet laser ablation system, the system comprising: a laser, a light homogenizing assembly, and an optical fiber combining assembly;
The laser is configured to emit laser light;
The light homogenizing assembly is disposed on the emergent light path of the laser light; the optical fiber combining assembly is disposed on the emergent light path of the light homogenizing assembly;
The light homogenizing assembly is configured to perform beam flat-top shaping and beam superposition on the laser light to obtain a target light spot;
The optical fiber combining assembly is configured to perform multi-beam transmission on the target light spot.

Optionally, the light homogenizing assembly comprises a diffractive optical element.

Optionally, the diffractive optical element is a holographic diffuser.

Optionally, the light homogenizing assembly comprises:
A micro-lens array, disposed on the emergent light path of the laser light, configured to perform beam flat-top shaping on the laser light to obtain a shaped beam;
A focusing lens, disposed on the emergent light path of the shaped beam, configured to perform beam superposition on the shaped beam to obtain the target light spot.

Optionally, the focusing lens is a plano-convex lens or a biconvex lens.
Optionally, the optical fiber combining assembly comprises: a large-core quartz optical fiber, a clustered input optical fiber, a clustered tapered optical fiber, and a bundled output optical fiber;
The large-core quartz optical fiber is disposed on the emergent light path of the light homogenizing assembly; the large-core quartz optical fiber is connected to the clustered tapered optical fiber with the interposition of the clustered input optical fiber; an output end of the clustered tapered optical fiber is connected to the bundled output optical fiber.

Optionally, the clustered input optical fiber comprises a plurality of small-core quartz optical fibers.

Optionally, the core diameter of the large-core quartz optical fiber is greater than 600 µm.

Optionally, the core diameter of the small-core quartz optical fiber is less than 200 µm.

Optionally, the optical fiber combining assembly comprises a protective sheath; the protective sheath covers the surface of the large-core quartz optical fiber.

According to the specific embodiments provided by the present invention, the present invention discloses the following technical effects:
The present invention provides an optical fiber combining-coupled ultraviolet laser ablation system, which includes a laser, a light homogenizing assembly, and an optical fiber combining assembly. The laser is configured to emit laser light; the light homogenizing assembly is disposed on the emergent light path of the laser light; the optical fiber combining assembly is disposed on the emergent light path of the light homogenizing assembly. The light homogenizing assembly is used to perform beam flat-top shaping and beam superposition on the laser light, and the optical fiber combining assembly is used to perform multi-beam transmission on the target light spot. By means of beam flat-top shaping and beam superposition of the light homogenizing assembly, the laser energy can be distributed and homogenized. Then, the optical fiber combining assembly performs multi-beam transmission on the target light spot, which increases the receiving area, thereby avoiding damage to the input end and achieving the purpose of uniformly receiving energy. Therefore, efficient and stable laser ablation can be realized.

### BRIEF DESCRIPTION OF THE DRAWINGS

To more clearly illustrate the embodiments of the present invention or the technical solutions in the prior art, the drawings required for use in the embodiments will be briefly introduced below. Obviously, the drawings in the following description are only some embodiments of the present invention. For those of ordinary skill in the art, other drawings can be obtained according to these drawings without creative labor.
FIG. 1 is a structural diagram of an optical fiber combining-coupled ultraviolet laser ablation system provided in an embodiment of the present invention;
FIG. 2 is a schematic diagram of an optical fiber combining assembly provided in an embodiment of the present invention;
FIG. 3 is a cross-sectional structural diagram of a clustered input optical fiber provided in an embodiment of the present invention;
FIG. 4 is a schematic diagram of a bundled output optical fiber provided in an embodiment of the present invention;
FIG. 5 is a schematic diagram of an ablation system in practical application.

Symbols: Laser-1, Light homogenizing assembly-2, Optical fiber combining assembly-3, Large-Core Quartz Optical Fiber-4, clustered input optical fiber-5, clustered tapered optical fiber-6, Bundled Output Optical Fiber-7, Quartz Capillary Tube-8, Small-Core Quartz Optical Fiber-9, Connector-10, Handle-11, Output Port-12, Guidewire-13, Micro-Lens Array-14, Focusing Lens-15.

### DETAILED DESCRIPTION

The technical solutions in the embodiments of the present invention will be clearly and completely described below with reference to the drawings in the embodiments of the present invention. Obviously, the described embodiments are only a part of the embodiments of the present invention, rather than all the embodiments. Based on the embodiments of the present invention, all other embodiments obtained by those of ordinary skill in the art without creative labor shall fall within the protection scope of the present invention.

The objective of the present invention is to provide an optical fiber combining-coupled ultraviolet laser ablation system, which enables efficient and stable laser ablation.

In order to make the above objectives, features, and advantages of the present invention more obvious and understandable, the present invention will be further described in detail below with reference to the drawings and specific embodiments.

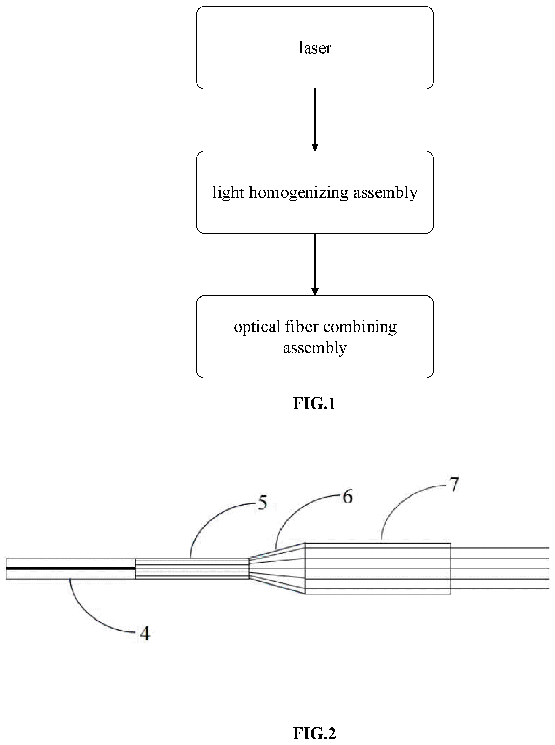

As shown in FIGS. 1-5, an optical fiber combining-coupled ultraviolet laser ablation system provided in an embodiment of the present invention includes: a laser 1, a light homogenizing assembly 2, and an optical fiber combining assembly 3.

The laser 1 is configured to emit laser light; the light homogenizing assembly 2 is disposed on the emergent light path of the laser light; the optical fiber combining assembly 3 is disposed on the emergent light path of the light homogenizing assembly 2; the light homogenizing assembly 2 is configured to perform beam flat-top shaping and beam superposition on the laser light to obtain a target light spot; the optical fiber combining assembly 3 is configured to perform multi-beam transmission on the target light spot.

Specifically, the light homogenizing assembly 2 includes a diffractive optical element. The diffractive optical element is a holographic diffuser.

In an embodiment, the light homogenizing assembly 2 includes a micro-lens array 14 and a focusing lens 15. The micro-lens array 14 is disposed on the emergent light path of the laser light; the focusing lens 15 is disposed on the emergent light path of the shaped beam. The focusing lens 15 is a plano-convex lens or a biconvex lens.

The micro-lens array 14 is configured to perform beam flat-top shaping on the laser light to obtain a shaped beam; the focusing lens 15 is configured to perform beam superposition on the shaped beam to obtain the target light spot.

The optical fiber combining assembly 3 includes a large-core quartz optical fiber 4, a clustered input optical fiber 5, a clustered tapered optical fiber 6, and a bundled output optical fiber 7.

The large-core quartz optical fiber 4 is disposed on the emergent light path of the light homogenizing assembly 2; the core diameter of the large-core quartz optical fiber 4 is greater than 600 µm.

The large-core quartz optical fiber 4 is connected to the clustered tapered optical fiber 6 with the interposition of the clustered input optical fiber 5; the output end of the clustered tapered optical fiber 6 is connected to the bundled output optical fiber 7. The clustered input optical fiber 5 includes a plurality of small-core quartz optical fibers 9. The core diameter of the small-core quartz optical fiber 9 is less than 200 µm.

In an embodiment, the optical fiber combining assembly 3 further includes a protective sheath; the protective sheath covers the surface of the large-core quartz optical fiber 4.

In practical application, the wavelength of the UV laser is selected to be in the range of 200 nm-400 nm. The light homogenizing assembly 2 is a coupling device for receiving UV laser energy, and the optical fiber combining assembly 3 is a conductor device for transmitting laser energy. When the laser passes through the array composed of the micro-lens array 14, the microlens array 14 performs beam flat-top shaping on the laser and splits the input laser light spot. Then, the subsequent focusing lens 15 superimposes multiple small beams of the split light spot, thereby obtaining a homogenized light field distribution. This reduces the laser energy intensity received per unit area at the fiber input end, thereby avoiding damage to the end face of the input end and forming a uniform target light spot.

The laser catheter effectively transmits laser energy from a single large-core optical fiber with a core diameter of more than 600 µm to multiple small-core optical fibers through the optical fiber combining assembly 3 with a core numerical aperture (NA) in the range of 0.22-0.50. Finally, a compact laser ablation system with uniform light spot distribution and integrated optical fiber coupling is realized.

The optical fiber combining assembly 3 is formed by connecting the large-core quartz optical fiber 4 to the clustered tapered optical fiber 6 with an interposition of the clustered input optical fiber 5 through fusion splicing process, and the clustered input optical fiber 5 includes a plurality of small-core quartz optical fibers 9. A quartz capillary tube 8 is used to bundle the plurality of small-core quartz optical fibers 9.

The clustered tapered optical fiber 6 is disposed in the quartz capillary tube 8, which effectively transmits laser energy from a single large-core optical fiber to a plurality of small-core quartz optical fibers 9. The core diameter of the small-core quartz optical fibers 9 is less than 200 µm. The optical fiber bundle composed of the plurality of small-core quartz optical fibers 9 includes an input section, a tapered section, and an output section. The diameters of the two ends of the tapered section are different. The large end of the tapered section is connected to the output section, and the small end of the tapered section is connected to the input section. The tapered section and the input section are both disposed in a front end of the quartz capillary tube 8, and the output section is located in a rear end of the quartz capillary tube 8 and extends out from the quartz capillary tube 8. The large-core quartz optical fiber 4 is disposed behind the focusing lens 15 in the optical path. The micro-lens array 14 homogenizes the laser energy emitted by the laser 1, and the large-core quartz optical fiber 4 receives the homogenized laser.

The pulse width of the laser 1 is less than 15 nanoseconds or sub-nanosecond, and the wavelength of the laser is from 200 nm to 400 nm; the light homogenizing assembly 2 may be provided with one or two micro-lens arrays; the light homogenizing assembly 2 may be a diffractive optical element; further, the light homogenizing assembly 2 may be a holographic diffuser. The focusing lens 15 is a plano-convex lens or a biconvex lens.

In addition, the protective sheath may cover the outer surfaces of the large-core quartz optical fiber 4 and the quartz capillary tube 8. The optical fiber combining assembly 3 may also be connected in the form of an SMA optical fiber connector. The large-core quartz optical fiber 4 is connected to one end of the optical fiber connector, and the clustered tapered optical fiber 6 is connected to the other end of the optical fiber connector. In addition, the optical fiber combining assembly 3 may be configured as a tapered optical fiber bundle only.

Current studies have found that the effective energy density of a UV laser ablation system needs to reach 40 mJ/mm²-60 mJ/mm². For high-peak-energy 355 nm UV laser with a nanosecond pulse width, the end face of the fiber input end is easily damaged, so that it is difficult to achieve optical fiber coupling. Usually, a large-core quartz optical fiber 4 is used as a conductor, however it cannot be used as a laser ablation catheter in blood vessels due to its large bending radius. A plurality of small-core quartz optical fibers 9 is used as the conductor of the catheter. Since the small-core optical fibers are flexible and have a smaller bending radius, they can be used for laser ablation in blood vessels.

With the optical fiber combining assembly 3 as the final conductor, the front end with a single large-core quartz optical fiber 4 can better transmit the homogenized laser energy to the small-core quartz optical fibers 9, and avoids directly damaging the end faces of the plurality of optical fibers.

In addition, the ablation optical fiber may be a single optical fiber with a relatively large core diameter, or an optical fiber bundle composed of a plurality of UV multimode optical fibers. The single large-core quartz optical fiber 4 has high structural strength and is not easy to break or be damaged during use; while the UV multimode optical fibers used in the optical fiber combining assembly 3 can effectively ensure the stability of its working performance. The single large-core quartz optical fiber and the optical fiber bundle have their own advantages and can be selected according to actual conditions.

The optical fiber beam-combining coupled UV laser ablation system provided by the present invention solves the problem of optical fiber coupling, and can use UV light with a wavelength of 266 nm or light with other common wavelengths (such as 1064 nm).

FIG. 2 is a schematic diagram of the optical fiber combining assembly, which includes a single large-core quartz optical fiber 4, a clustered input optical fiber 5, a clustered tapered optical fiber 6, and a bundled output optical fiber 7. The clustered input optical fiber 5 is composed of a plurality of small-core quartz optical fibers 9. The clustered tapered optical fiber 6 is located on the output side of the bundled input optical fiber 5. The tapered end face of the bundled input optical fiber 5 is spliced to one end of the large-core quartz optical fiber 4, and the bundled output optical fiber 7 serves as the output of the fiber beam-combining assembly 3. To improve the coupling efficiency between the bundled output optical fiber 7 and the large-core quartz optical fiber 4, it is preferable that the core diameter of the large-core quartz optical fiber 4 is approximately equal to the clustered core diameter of the clustered input optical fiber 5.

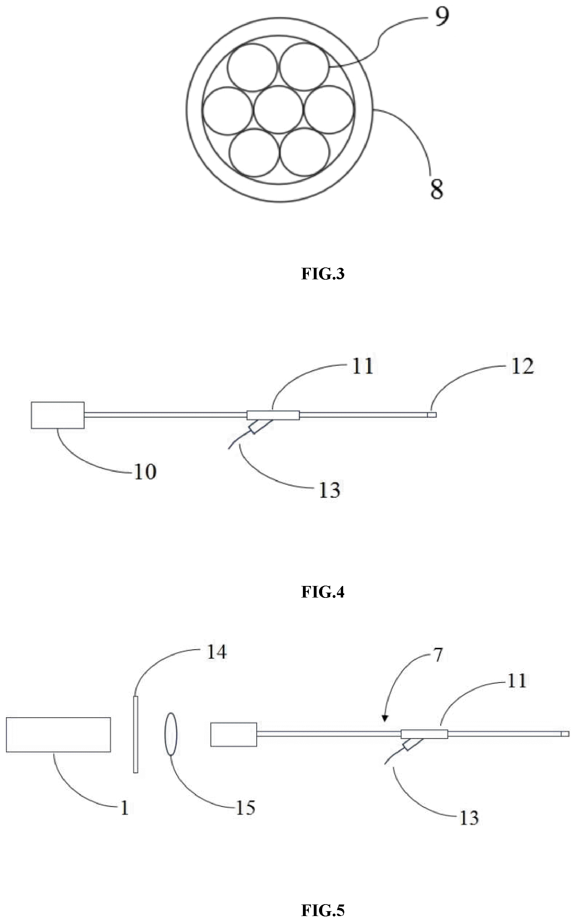

The clustered input optical fiber 5 includes a plurality of input optical fibers. In some embodiments, the plurality of input optical fibers are composed of a plurality of energy-transmitting optical fibers. As shown in the cross-sectional structural diagram of FIG. 3, the clustered input optical fiber 5 is composed of at least 7 energy-transmitting optical fibers, and a core diameter of the clustered input optical fiber 5 is less than 200 µm. The method for bundling the optical fibers mainly uses a quartz capillary tube 8 such as a circular capillary tube. The inner hole diameter of the circular capillary tube is 0.80 mm-1.00 mm, the outer diameter of the circular capillary tube is 1.05 mm-1.25 mm, and the length of the circular capillary tube is 100 mm-200 mm. After removing the coating layer of the plurality of small-core quartz optical fibers 9, they are disposed into a preset low-refractive-index fluorine-doped glass tube. In some embodiments, for example, after removing the coating layer from one end of seven optical fibers, the bare optical fibers are neatly arranged in a regular hexagon and all disposed into the low-refractive-index glass tube; the end face arrangement structure of the seven optical fibers after bundling is as shown in FIG. 3. After the optical fiber bundling is completed, fusion tapering is performed on a predetermined area on the input side of the clustered input optical fiber 5, and the length of the tapered area is determined to be 10 mm-30 mm (for example, 20 mm). The diameter of the output end interface is tapered to approximately 450 µm-750 µm (in some embodiments, 600 µm). The optical fiber bundle is tightly fixed and arranged together after high-temperature hot melting on a fusion splicer. After the fusion tapering is completed, a large-core optical fiber cleaver is used to cleave the tapered waist area, and the cleaved end face is as shown in FIG. 3. Then, a carbon dioxide laser fusion splicing method is used to splice the tapered end face of the cleaved optical fiber bundle with one end of the large-core quartz optical fiber 4.

FIG. 4 is a schematic diagram of the laser catheter according to the embodiment of the present invention, i.e., the bundled output optical fiber 7. The connector 10 of the laser catheter is provided with the aforementioned large-core quartz optical fiber 4. The laser catheter uses the large-core quartz optical fiber 4 as a connector, which can be conveniently connected to the light homogenizing assembly 2. Since no adhesive is used to fix the optical fibers in the front-end laser catheter, high-energy and short-pulse effective ablation treatment can be realized, and the thermal influence of the laser on human tissues is lower. The aforementioned optical fiber combining assembly 3 is disposed in the Y-shaped handle 11 of the laser catheter. The laser catheter uses the optical fiber combining assembly 3 as a conductor device to effectively transmit the laser light to the rear end of the laser catheter, which serves as the output port 12 of the laser catheter. As a possible implementation manner, an ablation optical fiber and a guidewire 13 is disposed in the inner cavity of the laser catheter. The ablation optical fiber is used to transmit the aforementioned target light spot, and the guidewire 13 can be controlled to guide the rear end of the laser catheter to a specified position in the blood vessel. The diameter of the ablation optical fiber is smaller than that of the guidewire 13, and the ablation optical fiber and the guidewire 13 are arranged noncoaxially with the catheter cavity (i.e., the ablation optical fiber and the guidewire 13 are arranged eccentrically in the catheter cavity).

FIG. 5 is a schematic diagram of the ablation system in practical application. Compared with the prior art, this optical fiber coupling system has a better effect: the single large-core quartz optical fiber 4 at the connector of the laser catheter (i.e., the bundled output optical fiber 7) has high structural strength and is not easy to break or be damaged during use; the UV multimode optical fibers used can ensure the stability of its working performance.

Advantages of the Present Invention are as follows:

In the coupled ultraviolet laser ablation system including the optical fiber combining assembly, the light homogenizing assembly and the optical fiber combining assembly are provided. When the laser light passes through the microlens array, the microlens array performs flat-top beam shaping on the laser light; subsequently, the focusing lens overlays the split light spots to form a uniform energy distribution. This reduces the laser energy intensity received per unit area at the incident end of the large-core quartz optical fiber. By distributing and homogenizing the laser energy and increasing the area for receiving the laser, damage to the end face of the incident end is avoided, and the purpose of uniformly applying energy is achieved.

The optical fiber combining assembly effectively transmits the homogenized laser energy from a single large-core quartz optical fiber to multiple small-core quartz optical fibers, avoiding direct damage to the end face of the incident end of the small-core quartz optical fibers. It also simplifies the entire fiber coupling and laser energy transmission system, enabling easy and effective laser ablation of tissues.

In the coupled ultraviolet laser ablation system with homogenization, the coupling efficiency of laser energy is improved, which reduces the requirement for the laser energy intensity of the laser. This allows the use of lasers with smaller volume and lighter weight, resulting in higher practicality.

The various embodiments in this specification are described in a progressive manner. Each embodiment focuses on explaining the differences from other embodiments, and the same or similar parts between the various embodiments may refer to each other.

Specific examples are used herein to illustrate the principles and implementations of the present invention. The descriptions of the above embodiments are only intended to help understand the system of the present invention and its core idea. Meanwhile, for those of ordinary skill in the art, based on the idea of the present invention, there will be changes in the specific implementations and application scopes. In conclusion, the content of this specification shall not be construed as a limitation on the present invention.

## Claims

1. An optical fiber combining-coupled ultraviolet laser ablation system, **characterized in that** the system comprises: a laser, a light homogenizing assembly, and an optical fiber combining assembly;
the laser is configured to emit laser light;
the light homogenizing assembly is disposed on the emergent light path of the laser light; the optical fiber combining assembly is disposed on the emergent light path of the light homogenizing assembly;
the light homogenizing assembly is configured to perform beam flat-top shaping and beam superposition on the laser light to obtain a target light spot;
the optical fiber combining assembly is configured to perform multi-beam transmission on the target light spot.

2. The optical fiber combining-coupled ultraviolet laser ablation system according to claim 1, **characterized in that** the light homogenizing assembly comprises: a diffractive optical element.

3. The optical fiber combining-coupled ultraviolet laser ablation system according to claim 2, **characterized in that** the diffractive optical element is a holographic diffuser.

4. The optical fiber combining-coupled ultraviolet laser ablation system according to claim 1, **characterized in that** the light homogenizing assembly comprises:
a micro-lens array, disposed on the emergent light path of the laser light, configured to perform beam flat-top shaping on the laser light to obtain a shaped beam;
a focusing lens, disposed on the emergent light path of the shaped beam, configured to perform beam superposition on the shaped beam to obtain the target light spot.

5. The optical fiber combining-coupled ultraviolet laser ablation system according to claim 4, **characterized in that** the focusing lens is a plano-convex lens or a biconvex lens.

6. The optical fiber combining-coupled ultraviolet laser ablation system according to claim 1, **characterized in that** the optical fiber combining assembly comprises: a large-core quartz optical fiber, a clustered input optical fiber, a clustered tapered optical fiber, and a bundled output optical fiber;
the large-core quartz optical fiber is disposed on the emergent light path of the light homogenizing assembly; the large-core quartz optical fiber is connected to the clustered tapered optical fiber with the interposition of the clustered input optical fiber; an output end of the clustered tapered optical fiber is connected to the bundled output optical fiber.

7. The optical fiber combining-coupled ultraviolet laser ablation system according to claim 6, **characterized in that** the clustered input optical fiber comprises a plurality of small-core quartz optical fibers.

8. The optical fiber combining-coupled ultraviolet laser ablation system according to claim 6, **characterized in that** the core diameter of the large-core quartz optical fiber is greater than 600 µm.

9. The optical fiber combining-coupled ultraviolet laser ablation system according to claim 7, **characterized in that** the core diameter of the small-core quartz optical fiber is less than 200 µm.

10. The optical fiber combining-coupled ultraviolet laser ablation system according to claim 6, **characterized in that** the optical fiber combining assembly comprises: a protective sheath;
the protective sheath covers the surface of the large-core quartz optical fiber.
